# EUROPEAN PATENT APPLICATION

(11) **EP 0 582 796 A1**
(43) Date of publication of application: **16.02.1994**
(21) Application number: 93108651.6
(22) Date of filing: 28.05.1993
(51) Int. Cl.: C12N 15/85, C07K 14/48, C12N 5/10, C12N 15/12

(54) **Promotor for expression and use thereof**

(30) Priority: 01.06.1992 JP 140706/92; 27.10.1992 JP 288717/92
(71) Applicant: Takeda Chemical Industries, Ltd., Osaka-shi, Osaka 541 (JP)
(72) Inventor: Shintani, Asae, Suita, Osaka 564 (JP); Sasada, Reiko, Nagaokakyo, Kyoto 617 (JP); Igarashi, Koichi, Kyoto 606 (JP)
(74) Representative: von Kreisler, Alek, Dipl.-Chem.

(57) **Abstract**

A novel DNA isolate encoding for a promoter region of human nerve growth factor-2, a recombinant vector containing said DNA, and a transformant carrying said recombinant vector are disclosed. The promoter of the present invention is useful as a means for analyzing an expression control mechanism of a nerve specific protein and can be used as a promoter which is incorporated into vectors used in the preparation of disease model animals, the treatment of diseases of organisms and the establishment of drug screening systems.

## Description

### FIELD OF THE INVENTION

The present invention relates to a novel promoter for gene expression and uses thereof. More particularly, the present invention relates to a recombinant DNA containing a promoter region of a nerve growth factor-2 (NGF-2) gene, a recombinant vector containing said DNA, and a transformant carrying said vector.

### PRIOR ART

In animals, particularly in higher animals, differentiation and maturation of various organs take place from the beginning of their development, which causes various functions of organisms to be exhibited. In this course, temporary or constant expression of proteins specific to various organs takes place, to give specificity to the organs. Genes of proteins which are specifically expressed and function at the fetal period or maturation period of development and differentiation have transcription induction systems such as promoters or enhancers which work only at that period, and the promoter portions control transcription of mRNA instructing protein synthesis.

Further, some promoters have been known to show hormone dependency or growth factor dependency. Drug screening systems and trans-genic mice have already been produced using these promoters, and have been utilized for drug screening or analysis of organisms.

Proteins belonging to neurotrophic factors family are nutritional factors essential for differentiation, growth and survival of nerve cells, and have hitherto been known as NGF (Leui-Monntalcini, Anu. N. Y. Acad. Sci., 55, 330 (1952)), polypeptide (I) (hereinafter referred as NGF-2) (European Paten Publication No. 386,752) and BDNF (J. Leibrock et al., Nature, 341, 149 (1989)). A peptide having the same amino acid sequence as NGF-2 is published under the name of NT-3 in A. Hohn et al., Nature, 344, 339 (1990) and is disclosed in PCT international Publication No. WO91/03569.

For NGF-2/NT-3, analysis of their mRNAs suggests that they start to be synthesized from the prenatal period and are most synthesized 1 to 2 weeks after birth (Y. Kaisho et al., Biochem. Biophy. Res. Commun., 174, 379 (1991)).

A transgenic animal can be prepared by injection of a DNA into an egg cell, where the DNA sequence has a promoter portion downstream from which structual genes for coding various proteins are bound, and wherein the promoter works only at a fetal period or maturation period of development and differrentiation. The thus prepared transgenic animals are useful to synthesize the proteins only at the fetal period or maturation period of development and differentiation and to study the function thereof in vivo. Further, the binding of an appropriate reporter gene to the above-mentioned promoter portion to establish a cell line which is capable of expressing the reporter gene allows the use thereof as a screening system for a drug having the function of promoting or depressing the synthesis of a protein essentially controlled by the promoter.

However, no clear expression time specificity is detected in almost all promoters instructing the transcription of various genes. A promoter exhibiting this specificity has therefore been earnestly desired.

A central nerve system is composed of nerve cells (neurons) differentiated from neural tubes and neurogliacytes (glia cells), and can be said to be a cell society performing complicated functions. A promoter which specifically functions in this system at a specific time of differentiation is useful as a means for analyzing an expression control mechanism of a nerve specific protein for functional differentiation.

Moreover, the introduction of a promoter, from which various genes are bound downstream, into a cell or an animal allows the genes to be specifically expressed in a nerve cell or in the brain at a specific time.

### SUMMARY OF THE INVENTION

For the purpose of discovering a promoter of an NGF-2 gene and taking out it as a promoter working at a specific time of development and differentiation, the present inventors cloned a human NGF-2/NT-3 genomic DNA, using a synthetic oligonucleotide corresponding to human NGF-2/NT-3 cDNA as a probe.

This gene was digested with a restriction enzyme to obtain a 4.0 kbp DNA fragment containing a portion of NGF-2/NT-3 cDNA and upstream portion thereof, and a 3.4 kbp upstream portion of the DNA fragment was subcloned on a plasmid DNA. A plasmid DNA was constructed in which the 3.4 kbp DNA was fused to a chloramphenicol acetyl transferase (CAT) gene as a reporter gene. CAT activity was measured of a transformant such as a glia cell or another animal cell transfected with the DNA, whereby an NGF-2/NT-3 promoter could be discovered in the 3.4-kb DNA of the upstream portion of an NGF-2/NT-3 structural gene.

As a result of further investigation based on these findings, the present inventors completed the present invention. According to the present invention, there are provided (1) a recombinant DNA comprising a promoter region of an NGF-2/NT-3 gene; (2) a recombinant vector comprising the recombinant DNA described in (1); (3) the recombinant vector described in (2), in which a structural gene is bound downstream from the promoter region; and (4) a transformant carrying the recombinant vector described in (2) or (3).

### BRIEF DESCRIPTION OF THE DRAWING

Figs. 1-1 and 1-2 show a nucleotide sequence from - 3275 to +546 containing the 5'-flanking region of the human NGF-2/NT-3 gene obtained in Example 1. In the figure, small letter code shows an intron and TATA box like sequence is enclosed with rectangle.

Fig. 2 shows the range of a 5'-flanking region of a human NGF-2/NT-3 gene contained in a plasmid used in Example 2.

Fig. 3 shows an autoradiogram of the CAT assay obtained in Example 2.

Fig. 4 shows an autoradiogram as a result of the CAT assay obtained in Example 3.

Fig. 5 is a partial schematic representation showing the construction of plasmid pTB1534 obtained in Example 4.

Fig. 6 is a completion of the schematic representation from Fig.5 showing the construction of the plasmid pTB1534 obtained in Example 4.

Fig. 7 is a cell growth curve obtained in Example 4.

Fig. 8 is a graph showing alkaline phosphatase activity tested in Example 4.

### DESCRIPTION OF THE PREFERED EMBODIMENTS

The promoter of the NGF-2/NT-3 gene described in (1) is preferably of human origin. Further, the promoter region preferably contains the following nucleotide sequence or a portion thereof: 
TGAGCGCGGA GCCATCTGGC CGGGTTGGCT GGTTATAACC GCGCAGATTC
TGTTCACGGG ACTC
(SEQ ID NO:1)
and more preferably contains the following sequence or a portion thereof: 
AAGAGGGGCC AGGAGAAATG ACCCCTTCCC CGCCACGGGT CCCGAAGTGA
GGGCGGGGGG GGGGCTCTGG GGCGCGGGCG CGCGCGGCGC GGCGCGGGCC
GGCGGGGGAG GGCGGCGCGG CGCGGAAGGG GTTAAGGCGC TGAGCGCGGA
 
GCCATCTGGC CGGGTTGGCT GGTTATAACC GCGCAGATTC TGTTCACGGG
ACTC
(SEQ ID NO:2)
The above-mentioned portions of the nucleotide sequences are portions having promoter activity.

The recombinant DNA of the present invention containing the NGF-2/NT-3 promoter region can be obtained for example, in the following manner.

First, using as a probe a synthetic oligonucleotide corresponding to 5'-noncoding region of human NGF-2/NT-3 cDNA reported in FEBS Lett., 266, 187-191 (1990), a human gene library was screened to obtain a clone of λ phage hybridize to this probe.

DNA is extracted from this phage clone, and a restriction enzyme map of a human gene portion incorporated therein is prepared. A DNA fragment hybridize to the probe, which is obtained, for example, by digestion with a restriction enzyme, can be subcloned, for example, into plasmid pUC118 [J. Vieira and J. Messing, Methods in Enzymology, 153, 3-11 (1987)]. The nucleotide sequence of the cloned DNA is determined and compared, for example, with the nucleotide sequence of the cDNA, whereby the position of a translation initiation codon on the gene can be found.

For example, the S1 mapping method [A. J. Berk and P. A. Sharp, Cell, 12, 721 (1977)] using human mRNA can find a transcription start site of the gene.

The recombinant vector comprises the recombinant DNA including the NGF-2/NT-3 promoter region thus obtained. Examples of vectors for recombination into which the NGF-2/NT-3 promoter is incorporated include but are not limited to vectors for animal cells such as the pCD vector, the cDM8 vector [A. Aruffo and B. Seed, Proc. Natl. Acad. Sic. USA, 84, 8573-8577 (1987)] and the retrovirus vector [R. D. Cone and R. C. Mulligan, Proc. Natl. Acad. Sci. USA, 81, 6349-6353 (1987); and vectors for Escherichia coli such as pUC [J. Vieira and J. Messing, Methods in Enzymology, 153, 3-11 (1987)].

The structural genes inserted downstream from the promoter regions in the recombinant vectors include structural genes coding for polypeptides for learning the functions in central nerve systems at the fetal period or maturation period of development and differentiation of various gene products.

Examples thereof include neurotrophic factors such as nerve cell growth factor (NGF), basic fibroblast growth factor (basic FGF) and acidic fibroblast growth factor (acidic FGF), other growth factors and lymphokines.

The above-mentioned structural genes also typically include at least one reporter gene. The β-galactosidase gene as well as the CAT gene and the alkaline phosphatase gene has been generally used as the above-mentioned reporter gene. However, any other structural gene can be used as long as a method for detecting their gene products is available.

In order to incorporate the above-mentioned structural gene into the vector, the structural gene is bound to an appropriate restriction enzyme-cleaved site existing downstream from the promoter region in a direction in which the gene is correctly transcribed.

As host cells transformed using the above-mentioned recombinant vectors, animal cells, particularly glia cells or cerebral nerve cells, can be used. Further, egg cells or ES cells [M. J. Evans and K. H. Kaufman, Nature, 292, 154 (1981)] may also be used in the course of DNA introduction into animal bodies.

Methods for transforming these cells include the calcium phosphate method [Graham et al., Virology, 52, 456 (1981)], the electroporation method [Ishizaki et al., Saibou Kougaku (Cell Technology), 5, 557 (1986)] and the microinjection method.

The use of the promoters of the present invention allows cells such as cerebral nerve cells to produce various polypeptides as described above.

Further, when oncogenes including myc and ras are used as the above-mentioned structural genes, the resulting vectors can be inserted into egg cells of animals such as mice, rats, dogs and cats to prepare disease model animals in which a specific cancer is induced at the fetal period or maturation period of development and differentiation of the cerebral nerve cells of the animals. Other heredopathia model animals can be prepared by the same transgenic method.

Furthermore, when the above-mentioned structural genes are ones coding for peptides useful in treating cerebral genetic diseases such as Alzheimer's disease and Parkinson disease, the vectors of the present invention are directly given to the brains of mammals such as mice, rats, dogs, cats and humans, or the vectors introduced into cultivated brain-derived cells are implanted in the brains, whereby the diseases can be treated.

Moreover, brain tumor can be treated by directly introducing a vectors into tumor cells, which vector is constructed using a cancer inhibitor gene as the structural gene.

In addition, to identify a compound which is capable of controlling, espesially activating the promoters of the present invention in cerebral nerve cells, it is possible to screen a compound which is capable of treating dementia by activating the promoters of the NGF-2/NT-3 genes in the brains to increase the amount of production of NGF-2/NT-3 in the brains.

It is also possible to know the control capability of the promoter activity of the compound by cultivating the above-mentioned transformants in the presence of the sample compound, and measuring the amounts of the gene products in broths.

The cultivation of the transformants is carried out by methods well known in the art. Examples of media include MEM medium containing about 5 to 20% fetal calf serum [Science, 122, 501 (1952)], DMEM medium [Virology, 8, 396 (1959)], RPMI 1640 medium [J. Am. Med. Assoc., 199, 519 (1967)] and 199 medium [Proc. Soc. Biol. Med., 73, 1 (1950)]. The pH is preferably about 6 to 8. The cultivation is usually carried out at about 30 to 40°C for about 15 to 60 hours with aeration or agitation if necessary.

The NGF-2/NT-3 promoter of the present invention is useful as a means for analyzing an expression control mechanism of a nerve specific protein and can be used as a promoter which is incorporated into vectors used in the preparation of disease model animals, the treatment of diseases of organisms and the establishment of drug screening systems.

When nucleotides, amino acids and so on are indicated by abbreviations in the specification and drawings, the abbreviations adopted by the IUPAC-IUB Commission on Biochemical Nomenclature or commonly used in the art are employed. For example, the following abbreviations are used. When the amino acids are capable of existing as optical isomers, it is understood that the L-forms are represented unless otherwise specified.
- DNA :: Deoxyribonucleic acid
- cDNA :: Complementary deoxyribonucleic acid
- A :: Adenine
- T :: Thymine
- G :: Guanine
- C :: Cytosine
- A or Ala :: Alanine
- C or Cys :: Cysteine
- D or Asp :: Aspartic acid
- E or Glu :: Glutamic acid
- F or Phe :: Phenylalanine
- G or Gly :: Glycine
- H or His :: Histidine
- I or Ile :: Isoleucine
- K or Lys :: Lysine
- L or Leu :: Leucine
- M or Met :: Methionine
- N or Asn :: Asparagine
- P or Pro :: Proline
- Q or Gln :: Glutamine
- R or Arg :: Arginine
- S or Ser :: Serine
- T or Thr :: Threonine
- V or Val :: Valine
- W or Trp :: Tryptophan
- Y or Tyr :: Tyrosine
Transformants E. coli DH1/pTB1518 and E. coli DH1/pTB1534 obtained in Example 2 described below were deposited with the Institute for Fermentation, Osaka, Japan (IFO) and the Fermentation Research Institute, the Agency of Industrial Science and Technology, the Ministry of International Trade and Industry (FRI), respectively. Their accession numbers and deposit dates are shown in Table 1.

**Table 1**

| | | |
|---|---|---|
| E. coli DH1/pTB1518 | IFO 15283 (April 16, 1992) | FERM BP-3849 (May 11, 1992) |
| E. coli DH1/pTB1534 | IFO 15383 (October 13, 1992) | FERM BP-4038 (October 19, 1992) |

### Example 1 Cloning of Human NGF-2/NT-3 Genomic DNA

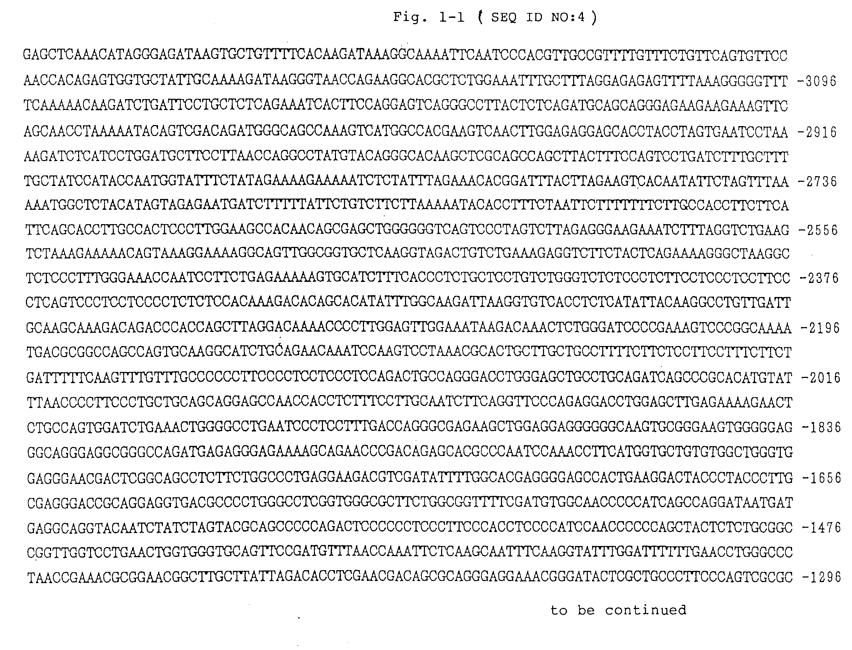

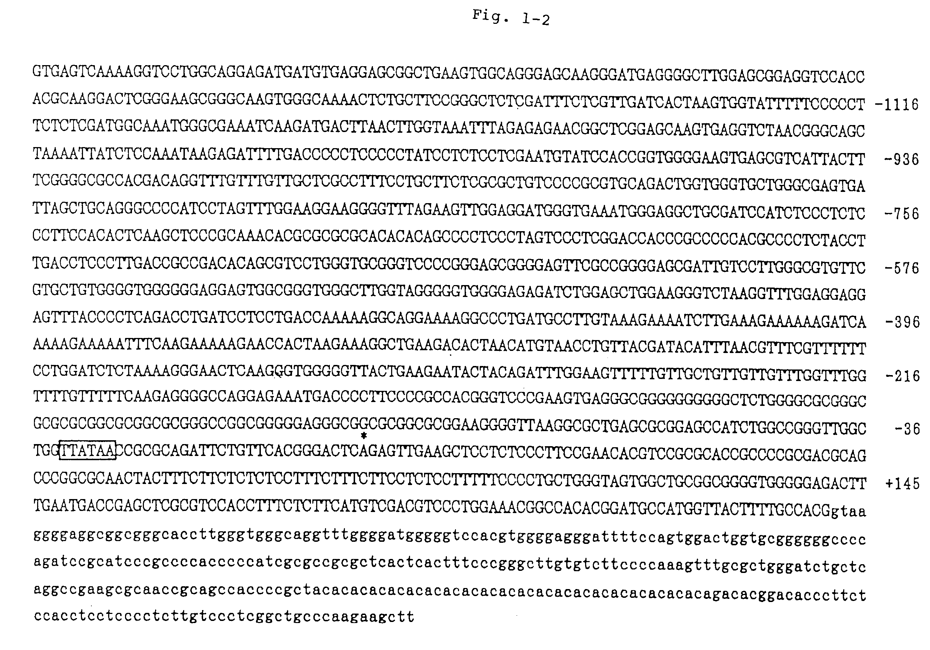

The oligonucleotide of 5'-TGCCATGGTTACTTTTGCCACG-3' (SEQ ID NO: 3) from nucleic acid residue Nos. 1 to 22 of NGF-2/NT-3 cDNA previously reported [Y. Kaisho et al., FEBS Lett., 266, 187-191 (1990)] was synthesized. Using this oligonucleotide as a probe, 2.3X10⁶ phages of the human genome libraries (Clontech, derived from human placental genomic DNA) were screened. Reaction was conducted in 5 X SSPE (1 X SSPE = 180 mM NaCl, 10 mM sodium phosphate, 1 mM EDTA; pH 7.7), 5 X Denhalt's solution (1 X Denhalt's solution = 0.02% polyvinylpyrrolidone, 0.02% Ficoll, 0.02% bovine serum albumin) and 100 µg/ml heat denatured salmon sperm DNA and 0.1% SDS, at 42°C for 16 hours. After reaction, the filter was washed with 6 X SSC (1 X SSC = 150 mM NaCl, 15 mM sodium citrate) at 45°C for 30 minutes, and further with 6 X SSC at 60°C for 30 minutes. Phages reactive to the probe were looked for by autoradiography of the filter. As a result, one positive clone could be obtained from 2.3X10⁶ phages. The Southern blotting hybridization revealed that an about 3.0-kbp BamHI-HindIII fragment hybridized with the probe. Then, the whole nucleotide sequence of this 3.0-kbp BamHI-HindIII and about 1.0 kbp upstream therefrom was determined. Namely, a nucleotide sequence of about 4.0 kbp containing the 5'-upstream region and the 3'-flanking region of the human NGF-2/NT-3 gene obtained here is shown in Figs. 1-1 and 1-2(SEQ ID NO:4). In the resulted sequence, a +210 to +231 portion of it has complete identity with the nucleotide sequence of human NGF-2/NT-3 cDNA. Further, a TATA box-like sequence (-32 to -27) is observed upstream from the 5'-noncoding region of cDNA, which indicates that the neighborhood of this region is a promoter region of the human NGF-2/NT-3 gene and a portion downstream from +232 is an intron.

### Example 2 Assay of Promoter Activity of Human NGF-2/NT-3 Gene

In order to confirm that the cloned genomic DNA fragment has promoter activity, the CAT assay method, one of the methods for testing promoter activity, was carried out.

In a plasmid having CAT as a reporter gene, the pCAT-Basic plasmid (Promega) was used as a vector. pCAT-Control plasmid (Promega) was used as a positive control.

The 5'-upstream region and the 3'-flanking region of the human NGF-2/NT-3 gene were deleted using a TAKARA Kilosequence deletion kit, and the deleted one was introduced between the HindIII site and the XbaI site upstream from the CAT gene of the vector.

Human glioma Hs683 cells were transfected with the plasmid by using the calcium phosphate method [M. Wigler et al., Cell, 16, 777 (1979)]. After transfected, cultivation was conducted in DMEM medium containing 10% fetal calf serum (FCS), and cells were collected after 48 hours. For the cell extract, CAT enzyme activity was measured. The cell extract was reacted in the presence of 5 mM acetyl CoA and 0.25 µCi ¹⁴C-chloramphenicol at 37°C for 4 hours, and then the reaction product was extracted with ethyl acetate. After ethyl acetate was evaporated to dryness, the residue was dissolved in 15 µl of ethyl acetate. Then, a thin-layer plate (Merck) was spotted with 7.5 µl of the solution. After development with developing solvents (CHCl₃/methanol: 95/1), an autoradiograph was taken.

First, the expression plasmid pTB1518 was constructed in which the 3.4-kbp DNA fragment from -3275 to +91 in Figs. 1-1 and 1-2 was inserted into the pCAT-Basic plasmid.

In order to store this plasmid pTB1518, E. coli DH1 was transformed using this plasmid to prepare the transformant E. coli DH1/pTB1518 (IFO 15283, FERM BP-3849).

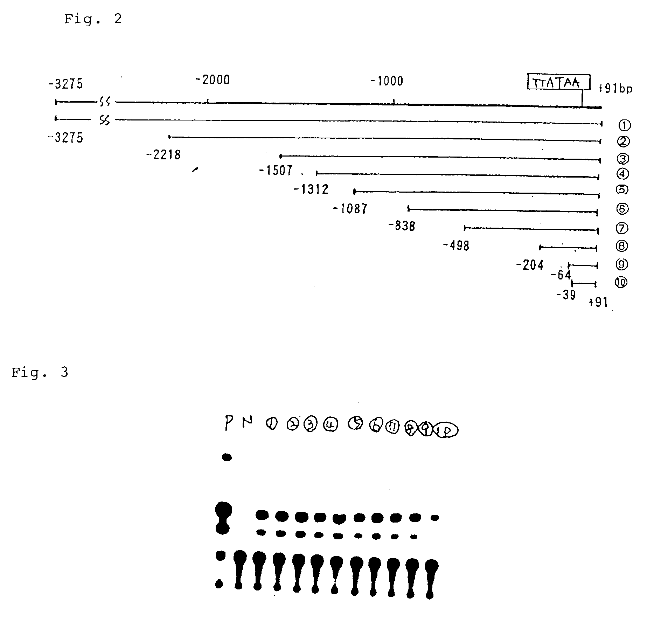

Using the above-mentioned plasmid pTB1518, the human glioma Hs683 cells were transformed to prepare the transformant human glioma Hs683/pTB1518. This transformant was cultivated in DMEM medium containing 10% FCS for 2 days, and thereafter cells were collected. The cell extract was assayed using the method of S. G. Widen et al. [J. Biol. Chem., 263, 16992-16998 (1988)]. Results thereof are shown in lane 1 in Figs. 2 and 3. Fig. 2 shows the fragments of the 5'-flanking region of the human NGF-2/NT-3 gene contained in the plasmid used, and Fig. 3 shows the autoradiograms resulting from the CAT assay.

As is shown in Fig. 3, CAT activity is clearly observed, similarlly as the CAT activity expressed under the domination of an SV40 initial promoter used as the positive control (shown as lane P in Fig. 3), and it was confirmed that a DNA fragment having functional promoter activity existed in this genomic DNA fragment.

Next, in order to know whether or not expression of activity is controlled according to the presence of a silencer or enhancer, various deletion mutants were constructed and subjected to a similar CAT assay.

Results thereof are also shown in Figs. 2 and 3. In Figs. 2 and 3, lanes 1 to 10 indicate results when pTB1518, pTB1519, pTB1520, pTB1521, pTB1522, pTB1523, pTB1524, pTB1525, pTB1526 and pTB1527 were each transfected. In this regard, pTB1519 is a vector in which the region from - 2218 to +91 is introduced into the pCAT-Basic plasmid, pTB1520 is a vector in which the region from -1507 to +91 is introduced, pTB1521 is a vector in which the region from -1312 to +91 is introduced, pBT1522 is a vector in which the region from -1087 to +91 is introduced, pTB1523 is a vector in which the region from -838 to +91 is introduced, pTB1524 is a vector in which the region from -498 to +91 is introduced, pTB1525 is a vector in which the region from - 204 to +91 is introduced, pTB1526 is a vector in which the region from -64 to +91 is introduced, and pTB1527 is a vector in which the region from -39 to +91 is introduced.

As is shown in Figs. 2 and 3, CAT activity is observed for the regions include region upstream from the region from -39 without significant difference from one another, whereas the activity was very low for the region from -39 to +91.

The above suggests that no clear silencer exists in the human NGF-2/NT-3 gene promoter, and that it is required to have at least the region from -64 to +91 to function as a promoter.

### Example 3 Assay of Promoter Activity of Human NGF-2/NT-3 Gene in Human Plasma-Derived ARH77 Cell

In order to examine host-specificity of the human NGF-2/NT-3 gene promoter, using cells in which no NGF-2/NT-3 genes were expressed, promoter activity thereof was measured. The following experiment was conducted by the Northern blotting hybridization using human plasma-derived ARH77 cells in which no NGF-2/NT-3 mRNA was confirmedly detected.

The ARH77 cells were transfected with plasmids by using the electroporation method [Ishizaki et al., Saibou Kougaku (Cell Technology), 5, 557 (1986)] under the conditions of 0.3 kv and 500 µF. After transfected, cultivation was conducted in RPMI-1640 medium containing 10% FCS, and cells were collected after 48 hours. For the cell extract, CAT enzyme activity was measured.

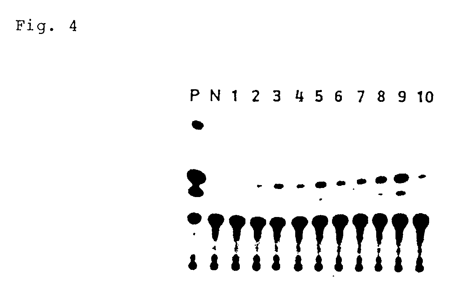

The plasmid employed in Example 2 was used as a plasmid having CAT as a reporter gene. Results of the CAT assay when each was transfected are shown in Fig. 4. As is shown in Figs. 2 and 4, the NGF-2/NT-3 promoters containing the regions upstream from -65 are very weak in CAT activity, and only the extract of cells transfected with pTB1526 (-64 to +91) exhibits a clear CAT activity. The above results suggest that the regions upstream from -65 include a region controlling the expression of the NGF-2/NT-3 gene.

### Example 4 Establishment of Human Glioma Hs683 Stable Strain into Which Human NGF-2 Promoter-Alkaline Phosphatase Fused Plasmid is Introduced

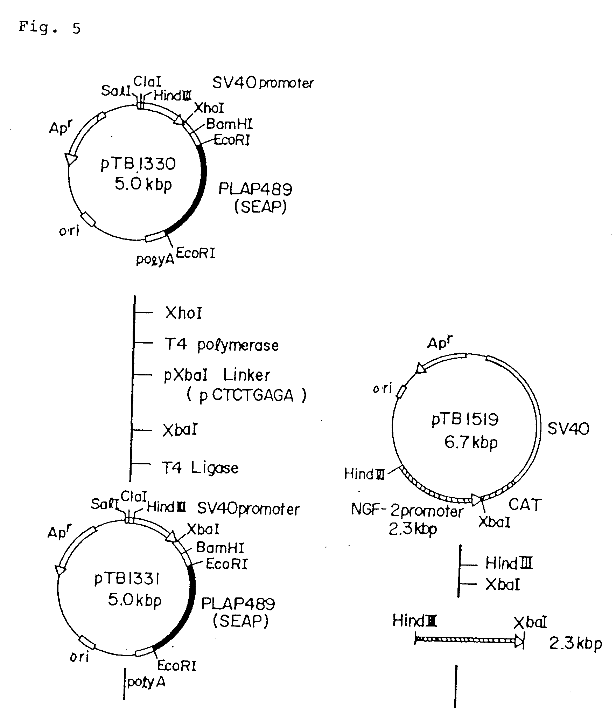

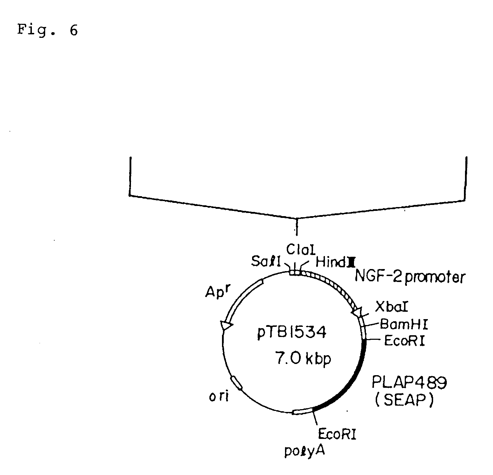

The construction of a plasmid having alkaline phosphatase as a reporter gene is shown in Figs. 5 and 6.

First, a 2.0-kbp EcoRI-KpnI fragment of pGem-4Z/PLAP*489 (SEAP) [J. Berger et al., Gene, 66, 1-10 (1988)] was isolated, and made flush with T4 DNA polymerase, followed by addition of the EcoRI linker (pGGAATTCC, Takara Shuzo). The resulting fragment was inserted into the EcoRI site of the vector pTB389 [Y. Ono et al., Science, 236, 1116-1120 (1987)] to construct pTB1330. pTB1330 was cleaved with XhoI, and made flush with T4 DNA polymerase, followed by addition of the XbaI linker (pCTCTGAGA) with T4 DNA ligase. Thus, pTB1331 was constructed.

Then, a 2.3-kbp NGF-2 promoter HindIII-XbaI fragment was isolated from pTB1519 obtained in Example 2, and inserted into the HindIII-XbaI site upstream from the alkaline phosphatase of pTB1331 constructed above to construct pTB1534. This was introduced into E. coli DH1 to prepare E. coli DH1/pTB1534 (IFO 15383, FERM BP-4038).

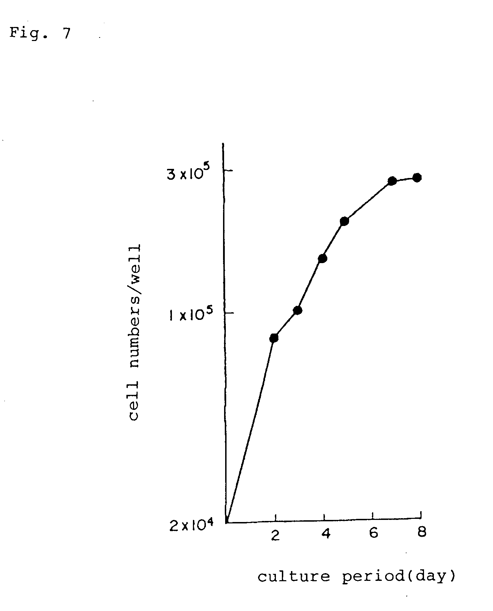

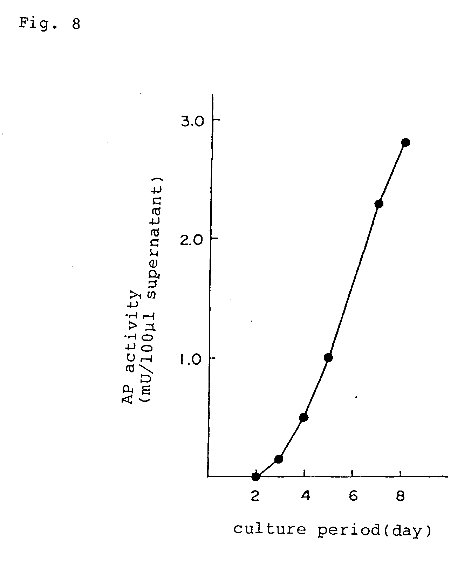

pTB6 having a neo gene [R. Sasada et al., Cell Structure and Function, 12, 205-217 (1987)] was used as a drug resistance marker. Human glioma Hs683 cells were concurrently infected with pTB1534 and pTB6, and after 48 hours after transfected, cultivation was conducted in DMEM medium containing 500 µg/ml G418 (O-2-amino-2,7-dideoxy-D-glycero-α-D-glucoheptopyranosyl[1→4]-O-3-deoxy-4C-methyl-3 [methylamino]-β-L-arabinopyranosyl-D-streptamine, Sigma) and 10% FCS to obtain a neomycin resistance strain. From the resulting neomycin resistance strain, one clone was obtained which stably expressed alkaline phosphatase. Alkaline phosphatase activity was measured as an increase in A₄₀₅ absorbance using p-nitrophenyl phosphate as a substrate. The culture supernatant was treated at 65°C for 5 minutes, followed by centrifugation at 12000 X g for 5 minutes. Then, 100 µl of the resulting supernatant was adjusted to a final amount of 200 µl with 1 X SEAP assay buffer (1.0 M diethanolamine, pH 9.8, 0.5 mM MgCl₂, 10 mM L-homoarginine). The solution was poured into a 98-well microtiter plate, and 20 µl of 120 mM p-nitrophenyl phosphate (in 1 X SEAP assay buffer) was added thereto, thereby measuring A₄₀₅. In this regard, 1 mU of alkaline phosphatase is taken as the amount of alkaline phosphatase which hydrolyzes 1 pmol of the substrate for 1 minute, which is represented by 1 mU = 0.4ΔA₄₀₅/min. For the resulting clone (Hs683/pTB1534), a growth curve of cells is shown in Fig. 7 and alkaline phosphatase activity in Fig. 8.

SEQUENCE LISTING

SEQ ID NO:1:

SEQUENCE LENGTH: 64 base pairs
SEQUNECE TYPE: nucleic acid
STRANDEDNESS: double
TOPOLOGY: linear

MOLECULE TYPE: DNA (genomic)

TGAGCGCGGA GCCATCTGGC CGGGTTGGCT GGTTATAACC GCGCAGATTC TGTTCACGGG 60

ACTC 64

SEQ ID NO:2:

SEQUENCE LENGTH: 204 base pairs
SEQUENCE TYPE: nucleic acid
STRANDEDNESS: double
TOPOLOGY: linear

MOLECULE TYPE: DNA (genomic)

AAGAGGGGCC AGGAGAAATG ACCCCTTCCC CGCCACGGGT CCCGAAGTGA GGGCGGGGGG 60

GGGGCTCTGG GGCGCGGGCG CGCGCGGCGC GGCGCGGGCC GGCGGGGGAG GGCGGCGCGG 120

CGCGGAAGGG GTTAAGGCGC TGAGCGCGGA GCCATCTGGC CGGGTTGGCT GGTTATAACC 180

GCGCAGATTC TGTTCACGGG ACTC 204

SEQ ID NO:3:

SEQUENCE LENGTH: 22 base pairs
SEQUENCE TYPE: nucleic acid
STRANDEDNESS: single
TOPOLOGY: linear

MOLECULE TYPE: Others, Synthetic nucleid acid

TGCCATGGTT ACTTTTGCCA CG 22

SEQ ID NO:4:
SEQUENCE LENGTH: 3821 base pairs

SEQUENCE TYPE: nucleic acid
STRANDEDNESS: double
TOPOLOGY: linear

MOLECULE TYPE: DNA (genomic)

ORIGINAL SOURCE:
(A) ORGANISM: Homo sapiens

FEATURE:
from 3276 to 3821 bp CDS
from 3507 to 3821 bp intron

GAGCTCAAAC ATAGGGAGAT AAGTGCTGTT TTCACAAGAT AAAGGCAAAA TTCAATCCCA 60

CGTTGCCGTT TTGTTTCTGT TCAGTGTTCC AACCACAGAG TGGTGCTATT GCAAAAGATA 120

AGGGTAACCA GAAGGCACGC TCTGGAAATT TGCTTTAGGA GAGAGTTTTA AAGGGGGTTT 180

TCAAAAACAA GATCTGATTC CTGCTCTCAG AAATCACTTC CAGGAGTCAG GGCCTTACTC 240

TCAGATGCAG CAGGGAGAAG AAGAAAGTTC AGCAACCTAA AAATACAGTC GACAGATGGG 300

CAGCCAAAGT CATGGCCACG AAGTCAACTT GGAGAGGAGC ACCTACCTAG TGAATCCTAA 360

AAGATCTCAT CCTGGATGCT TCCTTAACCA GGCCTATGTA CAGGGCACAA GCTCGCAGCC 420

AGCTTACTTT CCAGTCCTGA TCTTTGCTTT TGCTATCCAT ACCAATGGTA TTTCTATAGA 480

AAAGAAAAAT CTCTATTTAG AAACACGGAT TTACTTAGAA GTCACAATAT TCTAGTTTAA 540

AAATGGCTCT ACATAGTAGA GAATGATCTT TTTATTCTGT CTTCTTAAAA ATACACCTTT 600

CTAATTCTTT TTTTCTTCCC ACCTTCTTCA TTCAGCACCT TGCCACTCCC TTGGAAGCCA 660

CAACAGCGAG CTGGGGGGTC AGTCCCTAGT CTTAGAGGGA AGAAATCTTT AGGTCTGAAG 720

TCTAAAGAAA AACAGTAAAG GAAAAGGCAG TTGGCGGTGC TCAAGGTAGA CTGTCTGAAA 780

GAGGTCTTCT ACTCAGAAAA GGGCTAAGGC TCTCCCTTTG GGAAACCAAT CCTTCTGAGA 840

AAAAGTGCAT CTTTCACCCT CTGCTCCTGT CTGGGTCTCT CCCTCTTCCT CCCTCCTTCC 900

CTCAGTCCCT CCTCCCCTCT CTCCACAAAG ACACAGCACA TATTTGGCAA GATTAAGGTG 960

TCACCTCTCA TATTACAAGG CCTGTTGATT GCAAGCAAAG ACAGACCCAC CAGCTTAGGA 1020

CAAAACCCCT TGGAGTTGGA AATAAGACAA ACTCTGGGAT CCCCGAAAGT CCCGGCAAAA 1080

TGACGCGGCC AGCCAGTGCA AGGCATCTGC AGAACAAATC CAAGTCCTAA ACGCACTGCT 1140

TGCTGCCTTT TCTTCTCCTT CCTTTCTTCT GATTTTTCAA GTTTGTTTGC CCCCCTTCCC 1200

CTCCTCCCTC CAGACTGCCA GGGACCTGGG AGCTGCCTGC AGATCAGCCC GCACATGTAT 1260
TTAACCCCTT CCCTGCTGCA GCAGGAGCCA ACCACCTCTT TCCTTGCAAT CTTCAGGTTC 1320
CCAGAGGACC TGGAGCTTGA GAAAAGAACT CTGCCAGTGG ATCTGAAACT GGGGCCTGAA 1380
TCCCTCCTTT GACCAGGGCG AGAAGCTGGA GGAGGGGGGC AAGTGCGGGA AGTGGGGGAG 1440
GGCAGGGAGG CGGGCCAGAT GAGAGGGAGA AAAGCAGAAC CCGACAGAGC ACGCCCAATC 1500
CAAACCTTCA TGGTGCTGTG TGGCTGGGTG GAGGGAACGA CTCGGCAGCC TCTTCTGGCC 1560
CTGAGGAAGA CGTCGATATT TTGGCACGAG GGGAGCCACT GAAGGACTAC CCTACCCTTG 1620
CGAGGGACCG CAGGAGGTGA CGCCCCTGGG CCTCGGTGGG CGCTTCTGGC GGTTTTCGAT 1680
GTGGCAACCC CCATCAGCCA GGATAATGAT GAGGCAGGTA CAATCTATCT AGTACGCAGC 1740
CCCCAGACTC CCCCCTCCCT TCCCACCTCC CCATCCAACC CCCCAGCTAC TCTCTGCGGC 1800
CGGTTGGTCC TGAACTGGTG GGTGCAGTTC CGATGTTTAA CCAAATTCTC AAGCAATTTC 1860
AAGGTATTTG GATTTTTTGA ACCTGGGCCC TAACCGAAAC GCGGAACGGC TTGCTTATTA 1920
GACACCTCGA ACGACAGCGC AGGGAGGAAA CGGGATACTC GCTGCCCTTC CCAGTCGCGC 1980
GTGAGTCAAA AGGTCCTGGC AGGAGATGAT GTGAGGAGCG GCTGAAGTGG CAGGGAGCAA 2040
GGGATGAGGG GCTTGGAGCG GAGGTCCACC ACGCAAGGAC TCGGGAAGCG GGCAAGTGGG 2100
CAAAACTCTG CTTCCGGGCT CTCGATTTCT CGTTGATCAC TAAGTGGTAT TTTTCCCCCT 2160
TCTCTCGATG GCAAATGGGC GAAATCAAGA TGACTTAACT TGGTAAATTT AGAGAGAACG 2220
GCTCGGAGCA AGTGAGGTCT AACGGGCAGC TAAAATTATC TCCAAATAAG AGATTTTGAC 2280
CCCCTCCCCC TATCCTCTCC TCGAATGTAT CCACCGGTGG GGAAGTGAGC GTCATTACTT 2340
TCGGGGCGCC ACGACAGGTT TGTTTGTTGC TCGCCTTTCC TGCTTCTCGC GCTGTCCCCG 2400
CGTGCAGACT GGTGGGTGCT GGGCGAGTGA TTAGCTGCAG GGCCCCATCC TAGTTTGGAA 2460
GGAAGGGGTT TAGAAGTTGG AGGATGGGTG AAATGGGAGG CTGCGATCCA TCTCCCTCTC 2520
CCTTCCACAC TCAAGCTCCC GCAAACACGC GCGCGCACAC ACAGCCCCTC CCTAGTCCCT 2580
CGGACCACCC GCCCCCACGC CCCTCTACCT TGACCTCCCT TGACCGCCGA CACAGCGTCC 2640
TGGGTGCGGG TCCCCGGGAG CGGGGAGTTC GCCGGGGAGC GATTGTCCTT GGGCGTGTTC 2700
GTGCTGTGGG GTGGGGGGAG GAGTGGCGGG TGGGCTTGGT AGGGGGTGGG GAGAGATCTG 2760
GAGCTGGAAG GGTCTAAGGT TTGGAGGAGG AGTTTACCCC TCAGACCTGA TCCTCCTGAC 2820

CAAAAAGGCA GGAAAAGGCC CTGATGCCTT GTAAAGAAAA TCTTGAAAGA AAAAAGATCA 2880

AAAAGAAAAA TTTCAAGAAA AAGAACCACT AAGAAAGGCT GAAGACACTA ACATGTAACC 2940

TGTTACGATA CATTTAACGT TTCGTTTTTT CCTGGATCTC TAAAAGGGAA CTCAAGGGTG 3000

GGGGTTACTG AAGAATACTA CAGATTTGGA AGTTTTTGTT GCTGTTGTTG TTTGGTTTGG 3060

TTTTGTTTTT CAAGAGGGGC CAGGAGAAAT GACCCCTTCC CCGCCACGGG TCCCGAAGTG 3120

AGGGCGGGGG GGGGGCTCTG GGGCGCGGGC GCGCGCGGCG CGGCGCGGGC CGGCGGGGGA 3180

GGGCGGCGCG GCGCGGAAGG GGTTAAGGCG CTGAGCGCGG AGCCATCTGG CCGGGTTGGC 3240

TGGTTATAAC CGCGCAGATT CTGTTCACGG GACTC AGA GTT GAA GCT CCT CTC 3293
Arg Val Glu Ala Pro Leu
1               5

CCT TCC GAA CAC GTC CGC GCA CCG CCC CGC GAC GCA GCC CGG CGC AAC 3341
Pro Ser Glu His Val Arg Ala Pro Pro Arg Asp Ala Ala Arg Arg Asn
            10                  15                  20

TAC TTT CTT CTC TCT CCT TTC TTT CTT CCT CTC CTT TTT CCC CTG CTG 3389
Tyr Phe Leu Leu Ser Pro Phe Phe Leu Pro Leu Leu Phe Pro Leu Leu
        25                  30                  35

GGT AGT GGC TGC GGC GGG GTG GGG GAG ACT TTG AAT GAC CGA GCT CGC 3437
Gly Ser Gly Cys Gly Gly Val Gly Glu Thr Leu Asn Asp Arg Ala Arg
    40                  45                  50

GTC CAC CTT TCT CTT CAT GTC GAC GTC CCT GGA AAC GGC CAC ACG GAT 3485
Val His Leu Ser Leu His Val Asp Val Pro Gly Asn Gly His Thr Asp
55                  60                  65                  70

GCC ATG GTT ACT TTT GCC ACG GTA AGG GGA GGC GGC GGG CAC CTT GGG 3533
Ala Met Val Thr Phe Ala Thr Val Arg Gly Gly Gly Gly His Leu Gly
                75                  80                  85

TGG GCA GGT TTG GGG ATG GGG GTC CAC GTG GGG AGG GAT TTT CCA GTG 3581
Trp Ala Gly Leu Gly Met Gly Val His Val Gly Arg Asp Phe Pro Val
            90                  95                  100

GAC TGG TGC GGG GGG CCC CAG ATC CGC ATC CCG CCC CAC CCC CAT CGC 3629
Asp Trp Cys Gly Gly Pro Gln Ile Arg Ile Pro Pro His Pro His Arg
        105                 110                 115

GCC GCG CTC ACT CAC TTT CCC GGG CTT GTG TCT TCC CCA AAG TTT GCG 3677
Ala Ala Leu Thr His Phe Pro Gly Leu Val Ser Ser Pro Lys Phe Ala
    120                 125                 130

CTG GGA TCT GCT CAG GCC GAA GCG CAA CCG CAG CCA CCC CGC TAC ACA 3725
Leu Gly Ser Ala Gln Ala Glu Ala Gln Pro Gln Pro Pro Arg Tyr Thr
135                 140                 145                 150

CAC ACA CAC ACA CAC ACA CAC ACA CAC ACA CAC ACA CAG ACA CGG ACA    3773
His Thr His Thr His Thr His Thr His Thr His Thr Gln Thr Arg Thr
                155                 160                 165

CCC TTC TCC ACC TCC TCC CCT CTT GTC CCT CGG CTG CCC AAG AAG CTT    3821
Pro Phe Ser Thr Ser Ser Pro Leu Val Pro Arg Leu Pro Lys Lys Leu
            170                 175                 180

SEQ ID NO:5:

SEQUENCE LENGTH: 182 amino acids
SEQUENCE TYPE: amino acid
TOPOLOGY: linear

MOLECULE TYPE: protein

Arg Val Glu Ala Pro Leu Pro Ser Glu His Val Arg Ala Pro Pro Arg
  1               5                  10                  15

Asp Ala Ala Arg Arg Asn Tyr Phe Leu Leu Ser Pro Phe Phe Leu Pro
             20                  25                  30

Leu Leu Phe Pro Leu Leu Gly Ser Gly Cys Gly Gly Val Gly Glu Thr
         35                  40                  45

Leu Asn Asp Arg Ala Arg Val His Leu Ser Leu His Val Asp Val Pro
     50                  55                  60

Gly Asn Gly His Thr Asp Ala Met Val Thr Phe Ala Thr Val Arg Gly
 65                  70                  75                  80

Gly Gly Gly His Leu Gly Trp Ala Gly Leu Gly Met Gly Val His Val
                 85                  90                  95

Gly Arg Asp Phe Pro Val Asp Trp Cys Gly Gly Pro Gln Ile Arg Ile
            100                 105                 110

Pro Pro His Pro His Arg Ala Ala Leu Thr His Phe Pro Gly Leu Val
        115                 120                 125

Ser Ser Pro Lys Phe Ala Leu Gly Ser Ala Gln Ala Glu Ala Gln Pro
    130                 135                 140

Gln Pro Pro Arg Tyr Thr His Thr His Thr His Thr His Thr His Thr
145                 150                 155                 160

His Thr Gln Thr Arg Thr Pro Phe Ser Thr Ser Ser Pro Leu Val Pro
                165                 170                 175

Arg Leu Pro Lys Lys Leu
            180

## Claims

1. An isolated DNA encoding for a promoter region of human nerve growth factor 2.

2. A recombinant DNA containing a promoter region of human nerve growth factor 2.

3. The recombinant DNA according to claim 2, in which the promoter region contains the following nucleotide sequence or a portion thereof:

TGAGCGCGGA GCCATCTGGC CGGGTTGGCT GGTTATAACC GCGCAGATTC
TGTTCACGGG ACTC (SEQ ID NO:1).

4. The recombinant DNA according to claim 2, in which the promoter region contains the following nucleotide sequence or a portion thereof:

AAGAGGGGCC AGGAGAAATG ACCCCTTCCC CGCCACGGGT CCCGAAGTGA
GGGCGGGGGG GGGGCTCTGG GGCGCGGGCG CGCGCGGCGC GGCGCGGGCC
GGCGGGGGAG GGCGGCGCGG CGCGGAAGGG GTTAAGGCGC TGAGCGCGGA
GCCATCTGGC CGGGTTGGCT GGTTATAACC GCGCAGATTC TGTTCACGGG
ACTC (SEQ ID NO:2).

5. A recombinant vector containing the DNA according to claim 1 or 2.

6. The recombinant vector according to claim 5, in which a structural gene is incorporated downstream therefrom.

7. The recombinant vector according to claim 6 in which the structural gene is for a neurotrophic factor.

8. The recombinant vector according to claim 6 in which the structural gene is a reporter gene.

9. A host cell transformed with a recombinant vector according to claim 6.

10. A host cell according to claim 9 in which the host cell is an animal cell.
